# EUROPEAN PATENT SPECIFICATION

(11) **EP 0 601 664 B1**
(45) Date of publication and mention of the grant of the patent: **11.03.1998**
(21) Application number: 93203425.9
(22) Date of filing: 06.12.1993
(51) Int. Cl.: G01V 3/02

(54) **Measuring device for underground probing, and method for the application thereof**
Verfahren und Messvorrichtung zum Untersuchen des Untergrundes
Procédé et dispositif de mesure pour l'exploration du sous-sol

(30) Priority: 07.12.1992 NL 9202115
(43) Date of publication of application: 15.06.1994
(73) Proprietor: GRONDMECHANICA DELFT, 2628 CK Delft (NL)
(72) Inventor: de Feijter, Jan Willem, NL-2641 LN Pijnacker (NL)
(74) Representative: Smulders, Theodorus A.H.J., Ir.

(56) References cited:
- EP-A- 0 105 801
- US-A- 4 219 776

## Description

This invention relates to a measuring device for underground probing, comprising means suitable for transmitting and receiving electromagnetic waves, these means terminating in electrodes forming the free end of the measuring device, a soil sample being receivable in the measuring device between the electrodes.

A measuring device for underground probing is disclosed in European patent application 105801. This measuring device is lowered into a borehole filled with drilling fluid. The measuring device may also form part of a drill string, in which the measuring device is located precisely above the drill bit. The electromagnetic waves transmitted by a lower annular electrode of the measuring device are received by two annular electrodes located thereabove. The condition of the soil around the borehole is determined from the electrical measured values using an analyzing device arranged above the ground. In particular the electrical conductivity and the dielectric constant are thus determined.

The drawback of this measuring device resides in the fact that first a hole has to be drilled, in which the measuring device must be placed. The annular electrodes transmit signals, which are received after transmission through the soil present around the borehole. However, the structure of the soil around the borehole has undergone modifications as a result of the drilling operation, while moreover the transmitted signals must pass twice through a fluid layer filled with soil particles. As a result, the measured data obtained are less accurate.

U.S. Patent No. 4,219,776 discloses a measuring device for underground probing, in which the electrodes form the free end of the measuring device. This measuring device is lowered into a hole priorly drilled to a specified depth and driven into the soil at the bottom of the borehole. Then the electrical capacitance the resistance and possibly other parameters of the soil between the electrodes are measured. After completion of the measurements the measuring device is retracted from the ground. The soil sample hauled up between the electrodes is removed, whereafter the measuring device is ready for a next measurement in the hole after it has been drilled deeper or in a next borehole.

The drawback of this measuring device is that the measuring device must always be retracted from the ground before a new measurement at greater depth can be performed. As a result of the drilling operation, the structure of the soil at the bottom of the borehole has undergone modifications and the measured data obtained are less accurate.

The object of the present invention is to provide a measuring device which allows measurement at several depths at a single probing position without the measuring device having to be removed from the ground after each measurement, while moreover the measuring signals pass exclusively through the nearly undisturbed soil to be examined.

To that end, the measuring device according to the invention is characterized by the features as defined in claim 1.

In this manner, the structure of the soil sample received in the measuring device is hardly affected, if at all, and a soil sample can be received in the measuring device and be ejected again at any desired depth. Also, the measured volume and the distance between the electrodes are known, which increases the accuracy of the measured values.

Further elaborated embodiments of the measuring device according to the invention are described in the subclaims.

The measuring device according to the invention can be applied in two different ways for carrying out underground probes.

According to a first method,
- the measuring device is forced into the ground to a desired depth, the core and the outer jacket of the measuring device assuming a relative position such that the mouth of the outer electrode is closed by the end face of the insulator,
- subsequently the outer jacket is forced further into the ground relative to the core for receiving a soil sample between the electrodes,
- after the measurements on the soil sample have been performed, the core is shifted relative to the outer jacket, whereby the measured soil sample is ejected from the measuring device.

According to this method, a soil sample to be examined is first received in the measuring device at a specified depth, then measured and then removed from the measuring device.

According to a second method, the measuring device is forced into the ground to a desired depth, the core and the outer jacket assuming a relative position such that the end face of the insulator is spaced from the mouth of the outer electrode, whilst the soil sample received in the outer electrode, after being measured, is allowed to flow away laterally via openings and is replaced by a fresh soil sample as the measuring device is forced deeper into the ground. The soil sample to be examined is already received in the measuring device as soon as the measuring device has arrived at a desired depth, and is removed therefrom as the measuring device is being forced deeper into the ground. In this method, a soil sample is discharged through the openings in the wall of the outer electrode in accordance with a through-flow principle which enables semicontinuous underground probing.

Embodiments of the measuring device according to the invention will now be further explained with reference to the accompanying drawings. In the drawings:
Fig. 1 is a diagrammatic overview of a measuring arrangement;
Fig. 2 is a section of a first embodiment of a measuring device;
Fig. 3 is a section of a second embodiment of a measuring device;
Fig. 4 is a section taken on the line IV-IV of Fig. 3;
Fig. 5 is a section of a third embodiment of a measuring device; and
Fig. 6 is a section taken on the line V-V of the Figs. 4 and 5.

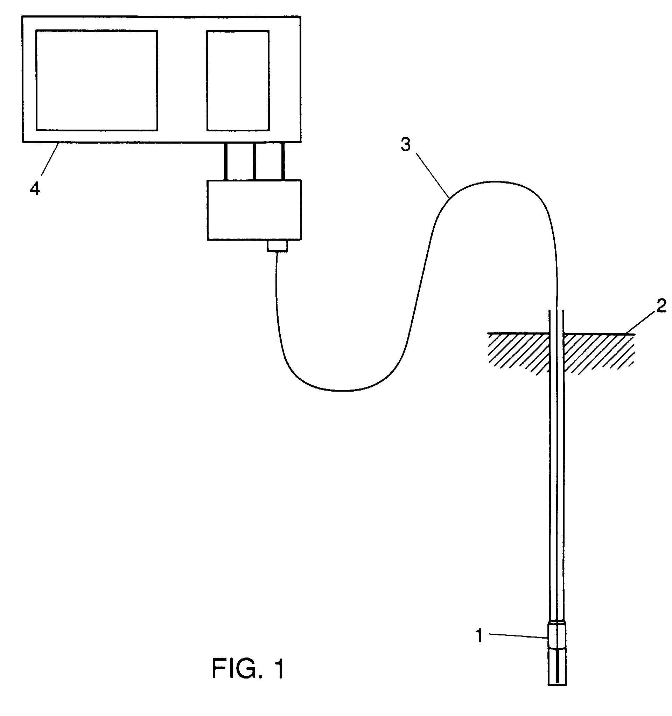

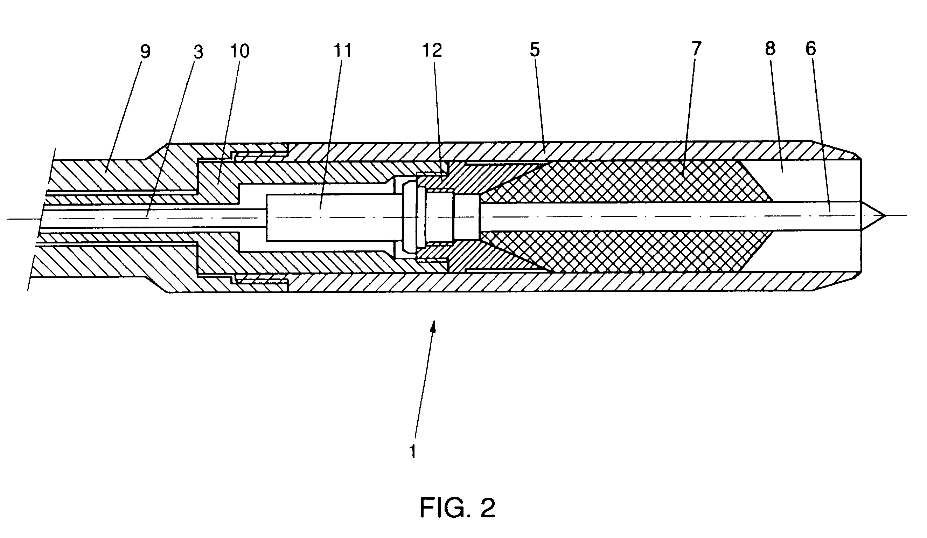

Fig. 1 diagrammatically shows a measuring device 1 driven into the ground 2. The measuring device 1 is connected to an analyzing device 4 via a coax cable 3 introduced into the measuring device 1 through a hollow core 10 (Fig. 2). Present around the core 10 is an outer jacket 9 which, together with the core 10, can be coupled to a probe rod (shown only diagrammatically in Fig. 1). Using this probe rod, the measuring device 1 can be driven into the ground 2, with the core 10 being movable relative to the outer jacket 9, i.e., being slidable in axial direction.

As shown in Fig. 2, the outer jacket 9 is connected to a hollow outer electrode 5, which has a bevelled open end. Coupled with the core 10 is a connecting piece 12 on which an insulator 7 and a rod-shaped inner electrode 6 are mounted. Both the insulator 7 and the rod-shaped inner electrode 6 have a pointed end, the free end of the inner electrode 6 projecting beyond the insulator 7 over a fixed distance. By sliding the core 10 and the outer jacket 9 relative to each other, the electrodes 5, 6 are also moved relative to each other. The relative displacement of the core 10 relative to the outer jacket 9 is limited by a mechanical stop. The electrode pair 5, 6 forms the transmission and reception device for the signals to be transmitted and received by the analyzing device 4.

The end of the coax cable 3 is coupled to the connecting piece 12 via a connector 11. The outer electrode 5 is electrically connected with the coax cable 3 via the connecting piece 12 and the connector 11. The inner electrode 6 is directly coupled with the core of the connector 11 and the outer electrode 5 is connected to the connecting piece 12 via a sliding contact. The insulator 7, too, is coupled with the connecting piece 12. The electrodes 5, 6 form as it were an extension of the coax cable 3. Thus, the measuring device 1 has optimum, low-reflection electromagnetic properties.

The outer jacket 9 and the core 10 are shifted relative to each other until the end of the insulator 7 and the bevelled end of the hollow outer electrode 5 lie substantially in the same plane and the inner electrode 6 projects outside the measuring device 1. In this position, the measuring device 1 is driven into the ground 2 using the probe rod. At the desired depth the core 10 is retained and the outer jacket 9 is forced slightly further into the ground 2, so that a soil sample 8 is received in the measuring device 1. After a measurement, to be discussed hereinafter, the soil sample 8 is pressed from the measuring device 1 owing to the core 10 being shifted further relative to the outer jacket 9 until the end of the insulator 7 and the bevelled end of the hollow electrode 5 lie substantially in the same plane. In this position the measuring device 1 is forced to a measuring point at a deeper location in the ground 2 and at that measuring point, by pressing the outer jacket 9 slightly further into the ground, a fresh soil sample 8 is received into the measuring device 1. Thereafter the measurement can be repeated.

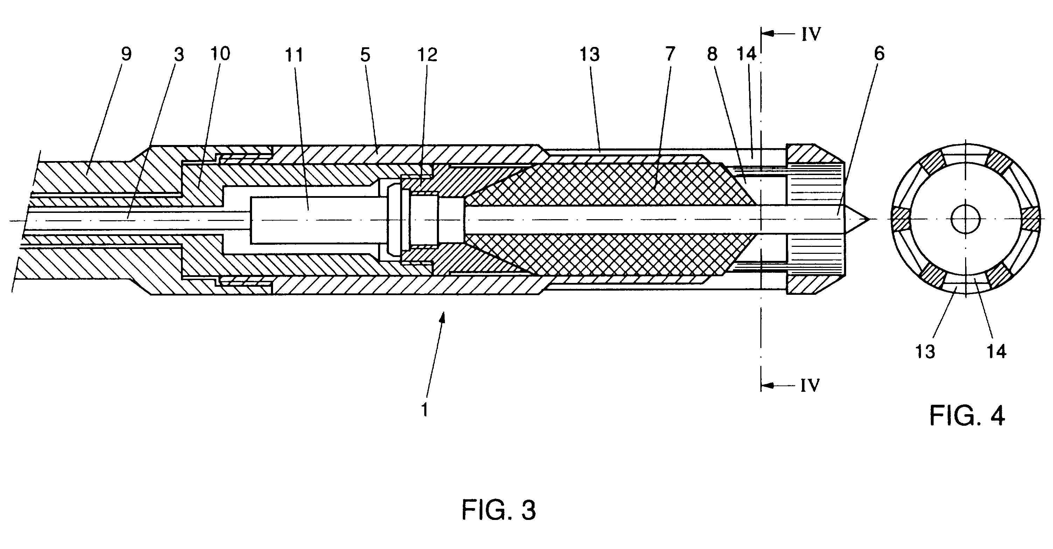

Further embodiments of the measuring device 1 are shown in Figs. 3-6. As shown in Fig. 3, slots 13 are provided in the outer surface of the outer electrode 5, which slots, at the level of the soil sample 8, merge into through-openings 14. This embodiment has the advantage that measurement can be done semicontinuously, owing to the fact that the outer jacket 9 and the core 10 - and hence the electrodes 5, 6 - need not be shifted relative to each other anymore. When the measuring device 1 is being driven into the ground 2, the inner electrode 6 (and the insulator 7) are in retracted position. As a result, the soil is discharged in upward direction along the pointed insulator 7 through the openings 14 and slots 13 in the outer electrode 5. The soil sample 8 is thus continuously refreshed owing to the soil passing through the measuring device 1. When the measuring device 1 has been brought to the desired measuring depth, measurement can take place immediately. Since the outer electrode 5 so designed is somewhat weaker than a closed electrode, this design is utilized for measurements in soil of slight strength.

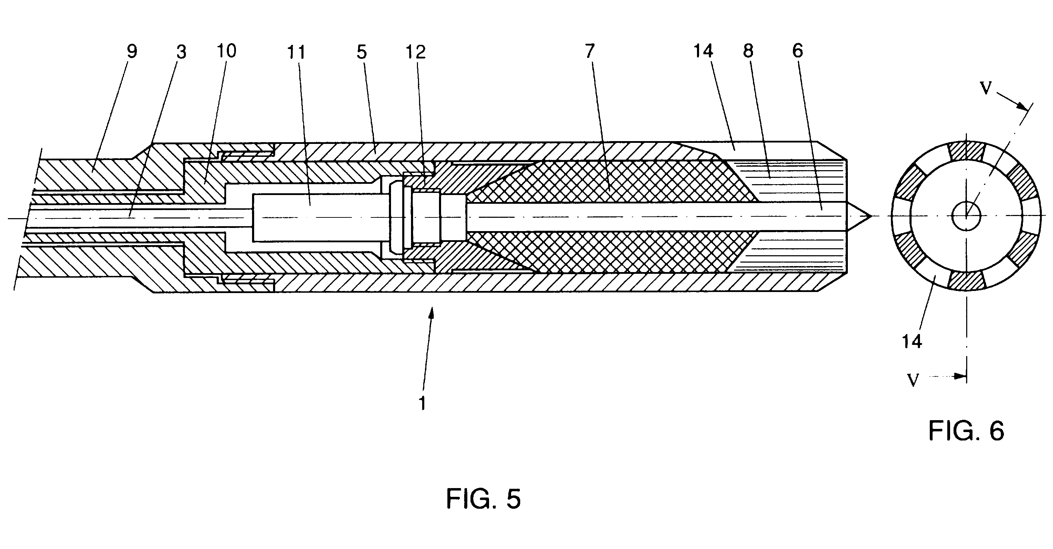

The outer electrode 5 according to Fig. 5 comprises slotted openings 14 extending up to the edge of the outer electrode 5. This type of measuring device 1 can be used in the same manner as the measuring device 1 according to Fig. 3. Since this embodiment is somewhat weaker still than the previous one, it is used for measurements in soft soil and/or at small depths. However, because the openings 14 extend as far as the edge of the outer electrode 5, the soil passes through the measuring device 1 more easily.

The parts that are subject to wear, such as the electrodes 5, 6 and the insulator 7, can be replaced easily and quickly.

In the embodiments of the outer electrode 5 according to Figs. 3 and 5, it may happen that the soil no longer flows through the measuring device but gets stuck between the electrodes 5, 6. By moving the electrodes 5, 6 relative to each other, the soil sample being stuck can be pressed from the measuring device 1.

In a different embodiment of the measuring device 1 (not shown), the electrodes 5, 6 are fixed relative to each other and only the insulator 7 is movable relative to the two electrodes 5, 6 for the purpose of receiving and ejecting a soil sample 8.

In this connection, in an underground probe according to the first method, the inner electrode 6 is incorporated into the insulator 6 and screened while the measuring device 1 is being forced into the ground.

With the measuring device 1 according to the invention, the electrical impedance of the soil is measured in a specified frequency range. The analyzing device converts the measured electrical impedance into electrical properties of the soil sample 8. Thus, as a function of the frequency, the relative dielectric constant and the electrical conductivity are determined. By the use of the measuring device 1 according to the invention, the interfering influences resulting from sampling are avoided in that the soil sample 8 is surrounded by the measuring electrodes 5, 6 and is replaced after the measurement.

The measuring device 1 can be introduced into the ground starting from a land surface, from a water surface, from the bottom under such water surface and from the bottom of a borehole. The measuring device can be simply adapted to all kinds of introducing devices. The depth range is limited only by the penetrative force of the introducing device and the mechanical strength of the measuring device. The measuring device can be introduced into all soft types of soil (whether or not saturated with water or other fluids) such as sand, clay, peat, humus and mixtures thereof.

## Claims

1. A measuring device for underground probing, comprising means suitable for transmitting and receiving electromagnetic waves, said means terminating in electrodes (5, 6) forming the free end of the measuring device (1), a soil sample (8) being receivable in the measuring device (1) between the electrodes (5, 6), characterized in that the outer electrode (5) has the shape of a hollow cylinder, which is mechanically connected to the end of an outer jacket (9), the inner electrode (6) being rod-shaped and connected to the end of a hollow core (10) via a connecting piece (12), while said outer jacket (9) and said hollow core (10) are capable of being forced directly into the ground (2) and are movable relative to each other in the axial direction for receiving a soil sample (8) between the electrodes (5, 6) and ejecting it respectively.

2. A measuring device according to claim 1, characterized in that the rod-shaped inner electrode (6) is surrounded by an insulator (7), the free end of the inner electrode (6) projecting a fixed distance outside the insulator (7).

3. A measuring device according to any one of claims 1-2, characterized in that the rod-shaped inner electrode (6) comprises a pointed end, while the hollow outer electrode (5) and the insulator (7) are bevelled at their free ends.

4. A measuring device according to any one of claims 1-3, characterized in that within the hollow core (10) a coax cable (3) is provided, which is electrically connected via a connector (11) with the inner electrode (6) and the outer electrode (5).

5. A measuring device according to any one of claims 1-4, characterized in that the outer electrode (5) is electrically connected via a sliding contact with the connecting piece (12) and the connector (11).

6. A measuring device according to any one of claims 1-5, characterized in that the outer jacket (9) and the core (10) are connectible to a probe rod for driving the measuring device (1) into the ground (2).

7. A measuring device according to any one of claims 1-6, characterized in that in the outer electrode (5), viewed in axial direction, adjacent the free end, slots (13) are provided which merge into openings (14) at the location of the soil sample (8).

8. A measuring device according to claim 7, characterized in that the openings (14) extend as far as the lower edge of the outer electrode (5).

9. A method for underground probing utilizing a measuring device according to any one of claims 1-6, characterized in that
- the measuring device (1) is forced into the ground (2) to a desired depth, the core (10) and the outer jacket (9) assuming a relative position such that the mouth of the outer electrode (5) is closed by the end face of the insulator (7),
- subsequently the outer jacket (9) is forced further into the ground (2) relative to the core (10) for receiving a soil sample (8) between the electrodes (5, 6),
- and that after the measurements on the soil sample have been performed, the core (10) is shifted relative to the outer jacket (9), whereby the measured soil sample is ejected from the measuring device (1).

10. A method for underground probing utilizing a measuring device according to any one of claims 7-8, characterized in that the measuring device (1) is forced into the ground (2) to a desired depth, the core (10) and the outer jacket (9) assuming a relative position such that the end face of the insulator (7) is spaced from the mouth of the outer electrode (5), whilst the soil sample (8) received in the outer electrode (5), after being measured, is allowed to flow away laterally via the openings (14) and is replaced by a fresh soil sample (8) as the measuring device (1) is forced deeper into the ground (2).

## Patentansprüche

1. Meßvorrichtung zur Untersuchung des Untergrundes, mit Mitteln, die geeignet sind zur Übertragung und Aufnahme von elektromagnetischen Wellen, wobei die genannten Mittel in Elektroden (5, 6) enden, die das freie Ende der Meßvorrichtung (1) bilden, wobei eine Bodenprobe (8) in der Meßvorrichtung (1) zwischen den Elektroden (5, 6) aufnehmbar ist,
dadurch gekennzeichnet,
daß die äußere Elektrode (5) die Form eines Hohlzylinders hat, der mechanisch mit dem Ende eines Außenmantels (9) verbunden ist, daß die innere Elektrode (6) stabförmig ist und mit dem Ende eines hohlen Kerns (10) über ein Verbindungsstück (12) verbunden ist, wobei der genannten Außenmantel (9) und der genannten hohle Kern (10) in der Lage sind, direkt in den Boden (2) eingetrieben zu werden und relativ zueinander in Axialrichtung beweglich sind, um eine Bodenprobe (8) zwischen den Elektroden (5, 6) aufzunehmen bzw. sie auszustoßen.

2. Meßvorrichtung gemäß Anspruch 1,
dadurch gekennzeichnet,
daß die stabförmige innere Elektrode (6) durch einen Isolator (7) umgeben ist, wobei das freie Ende der inneren Elektrode (6) um eine feste Strecke aus dem Isolator (7) hervorragt.

3. Meßvorrichtung gemäß einem der Ansprüche 1 bis 2,
dadurch gekennzeichnet,
daß die stabförmige innere Elektrode (6) ein spitzes Ende aufweist, während die hohle Außenelektrode (5) und der Isolator (7) an ihren freien Enden angefast sind.

4. Meßvorrichtung gemäß einem der Ansprüche 1 bis 3,
dadurch gekennzeichnet,
daß innerhalb des hohlen Kerns (10) ein Koaxialkabel (3) vorgesehen ist, das elektrisch über einen Verbinder (11) mit der inneren Elektrode (6) und der Außenelektrode (5) verbunden ist.

5. Meßvorrichtung gemäß einem der Ansprüche 1 bis 4,
dadurch gekennzeichnet,
daß die Außenelektrode (5) über einen Gleitkontakt mit dem Verbindungsstück (12) und dem Verbinder (11) elektrisch verbunden ist.

6. Meßvorrichtung gemäß einem der Ansprüche 1 bis 5,
dadurch gekennzeichnet,
daß der Außenmantel (9) und der Kern (10) mit einer Probenstange verbindbar sind, um die Meßvorrichtung (1) in den Boden (2) zu treiben.

7. Meßvorrichtung gemäß einem der Ansprüche 1 bis 6,
dadurch gekennzeichnet,
daß in der Außenelektrode (5) in Axialrichtung gesehen benachbart zu dem freien Ende Schlitze (13) vorgesehen sind, die an der Stelle der Bodenprobe (8) in Öffnungen (14) übergehen.

8. Meßvorrichtung gemäß Anspruch 7,
dadurch gekennzeichnet,
daß die Öffnungen (14) sich bis zur unteren Kante der Außenelektrode (5) erstrecken.

9. Verfahren zur Untersuchung des Untergrundes unter Benutzung einer Meßvorrichtung gemäß einem der Ansprüche 1 bis 6,
dadurch gekennzeichnet,
- daß die Meßvorrichtung (1) bis zu einer gewünschten Tiefe in den Boden (2) eingetrieben wird, wobei der Kern (10) und der Außenmantel (9) eine Relativstellung einnehmen, so daß die Mündung der äußeren Elektrode (5) durch die Endfläche des Isolators (7) geschlossen ist,
- daß nachfolgend der Außenmantel (9) relativ zum Kern (10) weiter in den Boden (2) eingetrieben wird, um eine Bodenprobe (8) zwischen den Elektroden (5, 6) aufzunehmen,
- und daß nachdem die Messungen an der Bodenprobe vorgenommen wurden, der Kern (10) relativ zu dem Außenmantel (9) verschoben wird, wodurch die gemessene Bodenprobe aus der Meßvorrichtung (1) ausgestoßen wird.

10. Verfahren zur Untersuchung des Untergrundes unter Benutzung einer Meßvorrichtung gemäß einem der Ansprüche 7 bis 8,
dadurch gekennzeichnet,
daß die Meßvorrichtung (1) bis zu einer gewünschten Tiefe in den Boden (2) eingetrieben wird, wobei der Kern (10) und der Außenmantel (9) eine relative Stellung einnehmen, so daß die Endfläche des Isolators (7) von der Mündung der äußeren Elektrode (5) beabstandet ist, während der Bodenprobe (8), die in der äußeren Elektrode (5) aufgenommen wird, nachdem sie gemessen wurde, ermöglicht wird, lateral über die Öffnungen (14) wegzufließen und sie durch eine frisch Bodenprobe (8) ersetzt wird, während die Meßvorrichtung (1) tiefer in den Boden (2) eingetrieben wird.

## Revendications

1. Appareil de mesure de sondage souterrain, comprenant un dispositif destiné à transmettre et recevoir des ondes électromagnétiques, le dispositif se terminant par des électrodes (5, 6) formant l'extrémité libre de l'appareil de mesure (1), un échantillon (8) de sol pouvant être logé dans l'appareil de mesure (1) entre les électrodes (5, 6), caractérisé en ce que l'électrode externe (5) a la forme d'un cylindre creux qui est raccordé mécaniquement à l'extrémité d'une gaine externe (9), l'électrode interne (6) ayant une forme de tige et étant connectée à l'extrémité d'un noyau creux (10) par une pièce (12) de connexion, alors que la gaine externe (9) et le noyau creux (10) peuvent être introduits à force directement dans le sol (2) et sont mobiles mutuellement dans la direction axiale pour le logement d'un échantillon (8) de sol entre les électrodes (5, 6) et son éjection respectivement.

2. Appareil de mesure selon la revendication 1, caractérisé en ce que l'électrode interne (6) en forme de tige est entourée d'un isolateur (7), l'extrémité libre de l'électrode interne (6) dépassant d'une distance fixe à l'extérieur de l'isolateur (7).

3. Appareil de mesure selon l'une quelconque des revendications 1 et 2, caractérisé en ce que l'électrode interne (6) en forme de tige possède une extrémité pointue, alors que l'électrode externe creuse (5) et l'isolateur (7) sont chanfreinés à leurs extrémités libres.

4. Appareil de mesure selon l'une quelconque des revendications 1 à 3, caractérisé en ce que, à l'intérieur du noyau creux (10), est disposé un câble coaxial (3) qui est connecté électriquement par un connecteur (11) à l'électrode interne (6) et à l'électrode externe (5).

5. Appareil de mesure selon l'une quelconque des revendications 1 à 4, caractérisé en ce que l'électrode externe (5) est connectée électriquement par un contact glissant à la pièce (12) de connexion et au connecteur (11).

6. Appareil de mesure selon l'une quelconque des revendications 1 à 5, caractérisé en ce que la gaine externe (9) et le noyau (10) peuvent être raccordés à une tige de sonde destinée à introduire l'appareil de mesure (1) dans le sol (2).

7. Appareil de mesure selon l'une quelconque des revendications 1 à 6, caractérisé en ce que, à l'intérieur de l'électrode externe (5) vue dans la direction axiale et près de l'extrémité libre, des fentes (13) sont disposées afin qu'elles rejoignent des ouvertures (14) à l'emplacement de l'échantillon (8) de sol.

8. Appareil de mesure selon la revendication 7, caractérisé en ce que les ouvertures (14) s'étendent jusqu'au bord inférieur de l'électrode externe (5).

9. Procédé de sondage souterrain mettant en oeuvre un appareil de mesure selon l'une quelconque des revendications 1 à 6, caractérisé en ce que
- l'appareil de mesure (1) est introduit à force dans le sol (2) à la profondeur voulue, le noyau (10) et la gaine externe (9) ayant une position relative telle que l'embouchure de l'électrode externe (5) est fermée par la face d'extrémité de l'isolateur (7),
- ensuite, la gaine externe (9) est introduite à force plus loin dans le sol (2) par rapport au noyau (10) pour le logement d'un échantillon (8) de sol entre les électrodes (5, 6), et
- après l'exécution des mesures de l'échantillon de sol, le noyau (10) est déplacé par rapport à la gaine externe (9), si bien que l'échantillon mesuré de sol est éjecté de l'appareil de mesure (1).

10. Procédé de sondage souterrain mettant en oeuvre un appareil de mesure selon l'une quelconque des revendications 7 et 8, caractérisé en ce que l'appareil de mesure (1) est introduit à force dans le sol (2) à une profondeur voulue, le noyau (10) et la gaine externe (9) ayant des positions relatives telles que la face d'extrémité de l'isolateur (7) est distante de l'embouchure de la seconde électrode (5), alors que l'échantillon (8) de sol, logé dans l'électrode externe (5) après sa mesure, peut s'écouler latéralement par les ouvertures (14) et est remplacé par un échantillon neuf (8) de sol lorsque l'appareil de mesure (1) est introduit à force plus profondément dans le sol (2).
